# EUROPEAN PATENT SPECIFICATION

(11) **EP 3 344 117 B1**
(45) Date of publication and mention of the grant of the patent: **02.10.2019**
(21) Application number: 16725192.5
(22) Date of filing: 08.04.2016
(51) Int. Cl.: A61B 3/16

(54) **DEVICE FOR MEASURING INTRA-OCULAR PRESSURE**
VORRICHTUNG ZUR MESSUNG DES INTRAOKULAREN DRUCKS
DISPOSITIF DE MESURE DE LA PRESSION INTRA-OCULAIRE

(30) Priority: 08.04.2015 GB 201505995
(43) Date of publication of application: 11.07.2018
(73) Proprietor: Aviram, David Paul, Kingston Upon Thames, Surrey KT1 3ET (GB)
(72) Inventor: Aviram, David Paul, Kingston Upon Thames, Surrey KT1 3ET (GB)
(74) Representative: Fry, David John
(86) International application number: PCT/GB2016/000077
(87) International publication number: WO 2016/162653

(56) References cited:
- WO-A2-2006/087715
- US-A- 4 628 938
- US-A- 5 032 020
- US-A1- 2002 159 031

## Description

The invention relates to a device for measuring Intra ocular pressure.

Measurement and detection of elevated Intra ocular pressures is a prime method for early detection of the onset of glaucoma.

Current methods for non-invasive detection of intra ocular pressures include the Goldman Aplanation method that involves the measurement of reactive forces due to a plunger, of known cross sectional area, engaging and flattening a small area of the cornea. Other methods involve the use of a jet of air for measuring the resulting corneal deformation. The main deficiencies of these methods arise from inaccuracies due to poor alignment with the radial axis of the cornea; the variation in local stiffness between cornea and the sclera and errors due to local deformation of a small area of the cornea when subjected to an external force.

Such constraints are known to have generated unreliable data about the actual pressure within the ocular region of the eye.

It is an aim of the present invention to provide an improved device for measuring inter ocular pressure. Another aim is to provide a solution of a type that is not made in a conventional manner and that will overcome the limitations mentioned above.

The invention provides a device for measuring intra ocular pressure as defined by claim 1 of the set of claims following this description.

Preferred and / or optional features of the invention are the subject of other claims of the set of claims.

An alternative solution for measuring intra ocular pressure that overcomes the above mentioned constraints is the main aim of this invention and will hereby be described by means of a non-limited example.

Accordingly, this invention provides for a pressure measuring device, characterized in that it has a pressure chamber that is equipped with a flexible membrane for contacting the cornea via an engagement contact lens and a strain measuring piece, on the opposite side of the pressure chamber, for measuring strains. Deflections of the cornea are dependent upon the difference between the applied external chamber pressure and the intraocular pressure. By using metered volumes of fluid to pressurise the chamber, a correlation between applied pressure and strains can be established. Hence, a comparison between strains and a range of applied pressures may be used to enable the determination of a specific range of inter ocular pressures. Results so obtained may be used to provide several data points that reduce errors associated with using a single data point as is the current practice. By such means it is possible to overcome errors due to directionality and local deformations, as mentioned above.

In one preferred embodiment of a pressure measuring device, a contact lens engagement piece is used for providing hygienic separation between the cornea and the flexible membrane of the pressure chamber. The strain measuring piece may be placed or form part of the flexible membrane and thus be directly affected by pressurisation from within the pressure chamber. In turn, deflections of the cornea will result in corresponding strains at the strain measuring piece. Thus, a correlation between strains and inter ocular pressure may be established.

One embodiment of the invention will be described solely by way of non-limited example and with reference to the accompanying drawings in which:
- **Fig. 1**: shows a general external view of a pressure measuring device in contact with an eye;
- **Fig. 2**: shows a sectional view of a pressure measuring device attached to the eye;
- **Fig. 3**: shows a sectional exploded view of a pressure measuring device near the eye;
- **Fig. 4**: shows a vertical section across the pressure chamber;

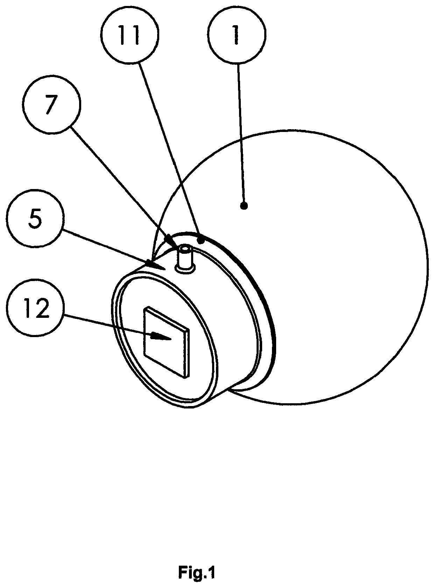

- **Fig. 1**: shows an assembly of a pressure measuring device. The assembly consists of pressure chamber (5), with external suction port (7) and strain measurement piece (12), attached to sclera (1) via engagement ring (11).

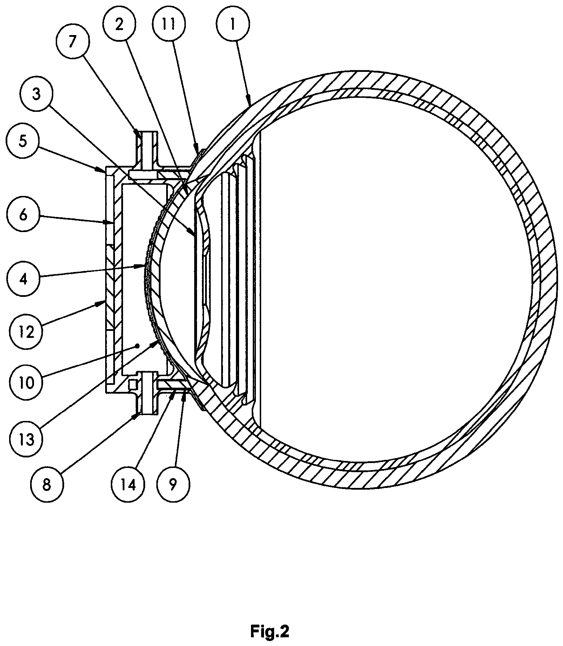

- **Fig. 2**: shows a vertical sectional view of an assembly of a pressure measuring device consisting of sclera (1), cornea (2) and regions of an eye. The pressure measuring device (5) has pressure chamber (10) that is equipped with strain measuring piece (12) and transparent piece (6). The pressure within the chamber (10) may be varied by means of pressure port (8). The flexible membrane (4) is held onto the sclera (1) by means of engagement ring (11) that has a peripheral suction channel (9), which is connected to an external suction port (7). The cornea (2) is in contact with contact lens engagement piece (13) that has ring (14) for axial alignment and engagement with peripheral suction channel (9). The contact lens engagement piece (13) is in contact with flexible membrane (4) and the cornea (2) which, in turn, is subjected to pressurisation from chamber (10). Transparent piece (6) enables investigation of the contours of the iris (3) by means of ultrasound and interferometry.

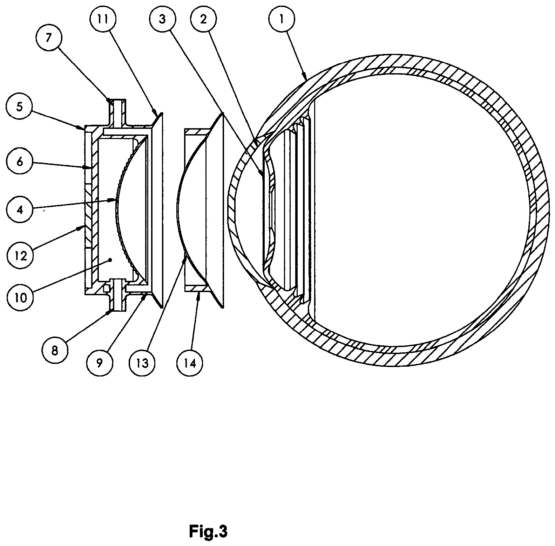

- **Fig.3**: shows a sectional exploded view of a pressure measuring device.

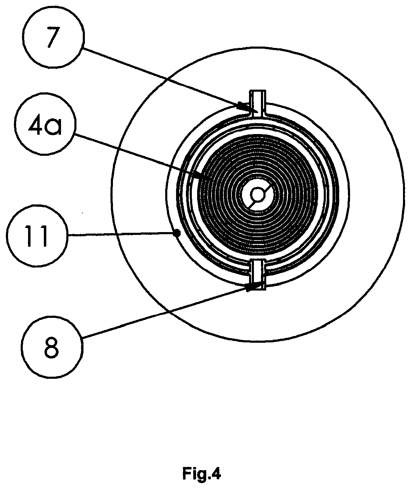

- **Fig.4**: shows a vertical section across the pressure chamber showing pressure port (8), suction port (7), strain gauge (4a) and engagement ring (11).

## Claims

1. 2. A device assembly for non-invasive determination of the intra-ocular pressure (IOP) of an eye, which has a cornea (2) bounded by a surrounding sclera (1), the device assembly comprising:
a pressure measuring device (5); and
a contact lens engagement piece (13);
said pressure measuring device (5) comprising:
a pressure chamber (10) with a flexible membrane (4);
an external suction port (7);
a pressure port (8);
an engagement ring (11) configured to be attached to the sclera (1); and
a strain measurement means (12);
wherein:
the pressure port (8) is in fluid contact with the pressure chamber (10) and is configured to charge the pressure chamber (10) with fluid to exert pressure on the cornea via the flexible membrane (4);
the pressure chamber (10) is equipped with said strain measurement means (12), said strain measurement means configured to determine the intra-ocular pressure due to the deflection of the cornea (2) by the exerted pressure;
wherein the flexible membrane (4) is held onto the sclera (1) by means of said engagement ring (11) wherein the contact between the flexible membrane (4) and the cornea (2) is enhanced by means of contact between the sclera (1) and the engagement ring (11) and wherein the engagement ring (11) is equipped with a peripheral suction channel (9) which is in fluid contact with the external suction port (7);
said contact lens engagement piece (13) has a ring (14) configured for axial alignment and engagement with the peripheral suction channel (9) of the engagement ring (11) and is located, in use, between the flexible membrane (4) and the cornea (2) and is in contact with said membrane (4) and cornea (2), said contact lens engagement piece (13) being configured to provide a hygienic separation between the cornea (2) and the flexible membrane (4).

2. A device assembly according to claim 1, wherein the flexible membrane (4) has a strain gauge (4a) attached to the outer surface of the flexible membrane (4) for determination of strains due to internal pressure variations within the pressure chamber (10).

3. A device assembly according to any previous claim wherein the pressure measuring device (5) has a transparent piece (6) which enables investigation of the contours of the iris (3) by means of ultrasound and interferometry.

## Patentansprüche

1. Vorrichtungsanordnung zur nicht-invasiven Bestimmung des Augeninnendrucks (intra-ocular pressure, IOD) eines Auges, das eine Hornhaut (2) aufweist, die durch eine umgebende Sklera (1) begrenzt ist, wobei die Vorrichtungsanordnung Folgendes umfasst:
eine Druckmessvorrichtung (5); und
ein Kontaktlinsen-Eingriffsteil (13);
wobei die Druckmessvorrichtung (5) Folgendes umfasst:
eine Druckkammer (10) mit einer flexiblen Membran (4);
einen externen Sauganschluss (7);
einen Druckanschluss (8);
einen Eingriffsring (11), der konfiguriert ist, um an der Sklera (1) angebracht zu werden; und
eine Dehnmesseinrichtung (12);
wobei:
der Druckanschluss (8) in Fluidkontakt mit der Druckkammer (10) ist und konfiguriert ist, um die Druckkammer (10) mit Fluid zu füllen, um über die flexible Membran (4) Druck auf die Hornhaut auszuüben;
die Druckkammer (10) mit der Dehnmesseinrichtung (12) ausgestattet ist, wobei die Dehnmesseinrichtung konfiguriert ist, um den Augeninnendruck aufgrund der Ablenkung der Hornhaut (2) durch den ausgeübten Druck zu bestimmen; wobei die flexible Membran (4) mittels des Eingriffsrings (11) an der Sklera (1) gehalten wird, wobei der Kontakt zwischen der flexiblen Membran (4) und der Hornhaut (2) mittels des Kontakts zwischen der Sklera (1) und dem Eingriffsring (11) verstärkt wird und wobei der Eingriffsring (11) mit einem peripheren Saugkanal (9) ausgestattet ist, der in Fluidkontakt mit dem externen Sauganschluss (7) ist;
das Kontaktlinsen-Eingriffsteil (13) einen Ring (14) aufweist, der für eine axiale Ausrichtung und einen Eingriff mit dem peripheren Saugkanal (9) des Eingriffsrings (11) konfiguriert ist, und sich in der Verwendung zwischen der flexiblen Membran (4) und der Hornhaut (2) befindet und mit der Membran (4) und der Hornhaut (2) in Kontakt ist, wobei das Kontaktlinsen-Eingriffsteil (13) konfiguriert ist, um eine hygienische Trennung zwischen der Hornhaut (2) und der flexiblen Membran (4) zu gewährleisten.

2. Vorrichtungsanordnung nach Anspruch 1, wobei die flexible Membran (4) einen Dehnmessstreifen (4a) aufweist, der zum Bestimmen von Dehnungen aufgrund von Innendruckschwankungen innerhalb der Druckkammer (10) an der Außenfläche der flexiblen Membran (4) angebracht ist.

3. Vorrichtungsanordnung nach einem vorherigen Anspruch, wobei die Druckmessvorrichtung (5) ein transparentes Teil (6) aufweist, das eine Untersuchung der Konturen der Iris (3) mittels Ultraschall und Interferometrie ermöglicht.

## Revendications

1. Ensemble dispositif pour une détermination non-invasive de la pression intra-oculaire (IOP) d'un oeil, qui possède une cornée (2) délimitée par une sclère entourante (1), l'ensemble dispositif comprenant :
un dispositif de mesure de pression (5) ; et
un élément d'engagement de lentille de contact (13) ;
ledit dispositif de mesure de pression (5) comprenant :
une chambre de pression (10) avec une membrane flexible (4) ;
un port d'aspiration externe (7) ;
un port de pression (8) ;
une bague d'engagement (11) configurée pour être attachée à la sclère (1) ; et
un moyen de mesure de contrainte (12) ;
dans lequel :
le port de pression (8) est en contact fluide avec la chambre de pression (10) et est configuré pour charger la chambre de pression (10) avec un fluide pour exercer une pression sur la cornée via la membrane flexible (4) ;
la chambre de pression (10) est équipée avec ledit moyen de mesure de contrainte (12), ledit moyen de mesure de contrainte configuré pour déterminer la pression intra-oculaire due à la déviation de la cornée (2) par la pression exercée ; dans lequel la membrane flexible (4) est maintenue sur la sclère (1) au moyen de ladite bague d'engagement (11) dans lequel le contact entre la membrane flexible (4) et la cornée (2) est amélioré au moyen d'un contact entre la sclère (1) et la bague d'engagement (11) et dans lequel la bague d'engagement (11) est équipée d'un canal d'aspiration périphérique (9) qui est en contact fluide avec le port d'aspiration externe (7) ;
ledit élément d'engagement de lentille de contact (13) possède une bague (14) configurée pour un alignement axial et un engagement avec le canal d'aspiration périphérique (9) de la bague d'engagement (11) et se situe, en utilisation, entre la membrane flexible (4) et la cornée (2) et est en contact avec lesdites membrane (4) et cornée (2), ledit élément d'engagement de lentille de contact (13) étant configuré pour fournir une séparation hygiénique entre la cornée (2) et la membrane flexible (4).

2. Ensemble dispositif selon la revendication 1, dans lequel la membrane flexible (4) possède une jauge de contrainte (4a) attachée à la surface externe de la membrane flexible (4) pour une détermination de contraintes dues à des variations de pression interne à l'intérieur de la chambre de pression (10).

3. Ensemble dispositif selon l'une quelconque revendication précédente, dans lequel le dispositif de mesure de pression (5) possède un élément transparent (6) qui permet une investigation des contours de l'iris (3) au moyen d'ultrasons et d'une interférométrie.
